# EUROPEAN PATENT SPECIFICATION

(11) **EP 2 889 034 B1**
(45) Date of publication and mention of the grant of the patent: **27.02.2019**
(21) Application number: 14196405.6
(22) Date of filing: 28.08.2009
(51) Int. Cl.: A61K 31/546, A61P 31/04

(54) **Composition comprising ceftaroline and tobramycin**
Zusammensetzungen enthaltend Ceftarolin und Tobramycin
Composition comprenant ceftaroline et tobramycine

(30) Priority: 28.08.2008 US 92497 P
(43) Date of publication of application: 01.07.2015
(62) Divisional of application: 09810635.4
(73) Proprietor: Pfizer Anti-Infectives AB, SE-191 90 Sollentuna (SE)
(72) Inventor: Biek, Donald, Mountain View, CA California 94040 (US)
(74) Representative: Pfizer

(56) References cited:
- EP-A1- 1 310 502
- EP-A1- 1 618 894
- SCHAADT R ET AL: "The In Vitro Activity of Ceftaroline in Combination with Other Antibacterial Agents", ABSTRACTS OF THE INTERSCIENCE CONFERENCE ON ANTIMICROBIAL AGENTS AND CHEMOTHERAPY, vol. 47, 2007, page 190, XP009164557, & 47TH INTERSCIENCE CONFERENCE ON ANTIMICROBIAL AGENTS AND CHEMOTHERAPY; CHICAGO, IL, USA; SEPTEMBER 17 -20, 2007 ISSN: 0733-6373
- BASSETTI MATTEO ET AL: "Novel beta-lactam antibiotics and inhibitor combinations.", EXPERT OPINION ON INVESTIGATIONAL DRUGS MAR 2008 LNKD- PUBMED:18321228, vol. 17, no. 3, March 2008 (2008-03), pages 285-296, XP002686830, ISSN: 1744-7658
- YAHAV ET AL: "Efficacy and safety of cefepime: a systematic review and meta-analysis", LANCET INFECTIOUS DISEASES, ELSEVIER LTD, US, vol. 7, no. 5, 1 May 2007 (2007-05-01), pages 338-348, XP022035509, ISSN: 1473-3099, DOI: 10.1016/S1473-3099(07)70109-3
- PARISH D ET AL: "Ceftaroline fosamil, a cephalosporin derivative for the potential treatment of MRSA infection", CURRENT OPINION IN INVESTIGATIONAL DRUGS, CURRENT DRUGS, LONDON, GB, vol. 9, no. 2, 1 February 2008 (2008-02-01), pages 201-209, XP008144501, ISSN: 0967-8298
- LENA M. NAPOLITANO: "Early Appropriate Parenteral Antimicrobial Treatment of Complicated Skin and Soft Tissue Infections Caused by Methicillin-Resistant Staphylococcus aureus", SURGICAL INFECTIONS, vol. 9, no. s1, 1 August 2008 (2008-08-01) , pages s17-s27, XP055210721, ISSN: 1096-2964, DOI: 10.1089/sur.2008.063.supp
- ATS BOARD DIRECTORS ET AL: "Guidelines for the management, of adults with hospital-acquired, ventilator-associated, and healthcare-associated pneumonia", AMERICAN JOURNAL OF RESPIRATORY AND CRITICAL CARE MEDICINE, vol. 171, no. 4, 15 February 2005 (2005-02-15), pages 388-416, XP002743928, ISSN: 1073-449X

## Description

### FIELD OF THE INVENTION

The present invention relates to compositions comprising ceftaroline fosamil, or a pharmaceutically acceptable salt or solvate thereof, in combination with tobramycin, or a pharmaceutically acceptable salt thereof.

### BACKGROUND OF THE INVENTION

Ceftaroline is a novel parenteral cephalosporin with a broad spectrum of activity against clinically important community-acquired and hospital-acquired Gram-negative and Gram-positive pathogens including methicillin-resistant *Staphylococcus aureus* and multidrug-resistant *Streptococcus pneumoniae.*

U.S. Patent No. 6,417,175 discloses compounds having excellent antibacterial activities for a broad range of Gram-positive and Gram-negative bacteria. These compounds are represented by the general formula: 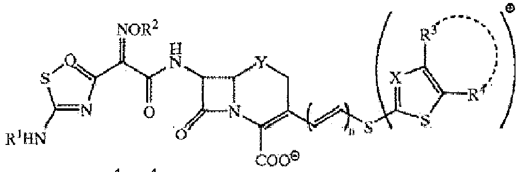 wherein R¹-R⁴, Q, X, Y and n are as defined therein.

U.S. Patent No. 6,417,175 discloses methods for preparing the compounds, and generically discloses formulations of the compounds, such as aqueous and saline solutions for injection. One such compound is 7β-[2(Z)-ethoxyimino-2-(5-phosphonoamino-1,2,4-thiadiazole-3-yl)acetamido]-3-[4-(1-methyl-4-pyridinio)-2-thiazolythio]-3-cephem-4-carboxylate.

U.S. Patent No. 6,906,055 discloses a chemical genus which includes compounds of formula:

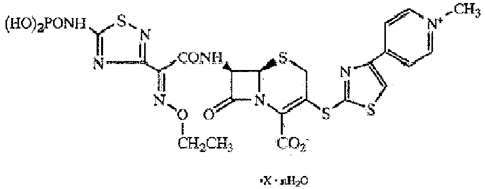

Ceftaroline fosamil is a sterile, synthetic, parenteral prodrug cephalosporin antibiotic. The N-phosphonoamino water-soluble prodrug is rapidly converted into the bioactive ceftaroline, which has been demonstrated to exhibit antibacterial activity.

Ceftaroline fosamil is known as (6*R*,7*R*)-7-{(2*Z*)-2-(ethoxyimino)-2-[5-(phosphonoamino)-1,2,4-thiadiazol-3-yl]acetamido}-3-{[4-(1-methylpyridin-1-ium-4-yl)-1,3-thiazol-2-yl]sulfanyl}-8-oxo-5-thia-1-azabicyclo[4.2.0]oct-2-ene-2-carboxylate. Ceftaroline fosamil may be an acetic acid hydrous form.

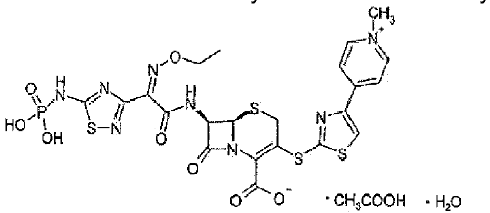

EP 1 310 502 A1 discloses the use of ceftaroline fosamil monoacetate monohydrate in the treatment of bacterial infections.

There remains a need in the art for new and improved compositions and methods directed to the treatment of bacterial infections.

It has been surprisingly and unexpectedly found that ceftaroline in combination with various antibacterial agents acts synergistically against bacterial strains. Furthermore, the combination of ceftaroline and antibacterial agents does not show evidence of antagonism. Thus, the findings suggest that ceftaroline may be suitable for administration in combination with one or more antibacterial agents.

### SUMMARY OF THE INVENTION

The present invention provides compositions comprising a therapeutically effective amount of ceftaroline fosamil, or a pharmaceutically acceptable salt or solvate thereof, in combination with tobramycin, or a pharmaceutically acceptable salt thereof.

### BRIEF DESCRIPTION OF THE DRAWINGS

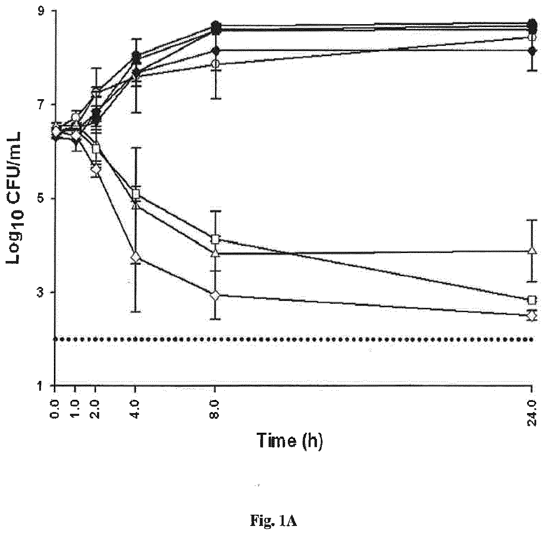

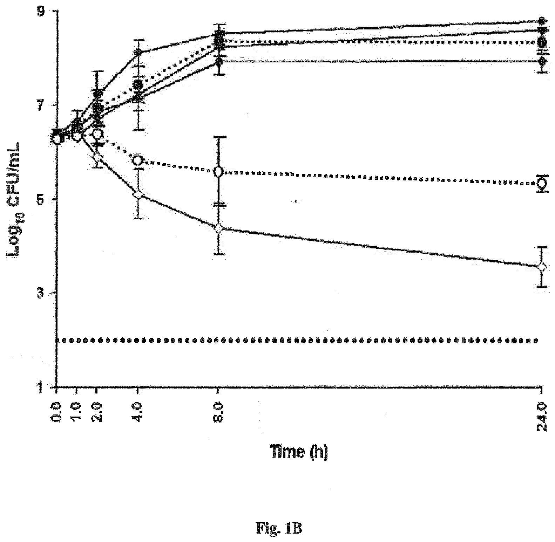

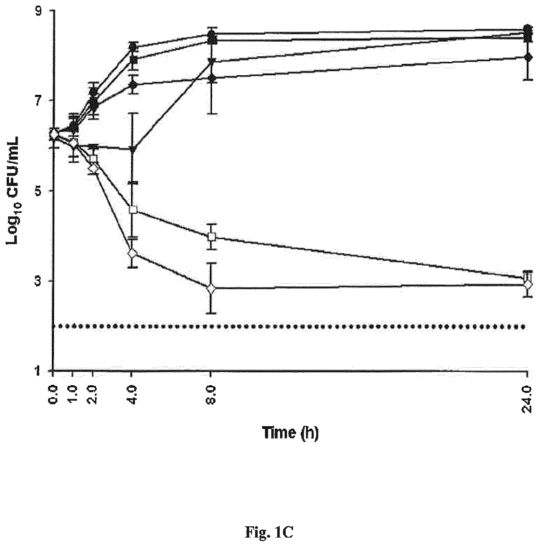

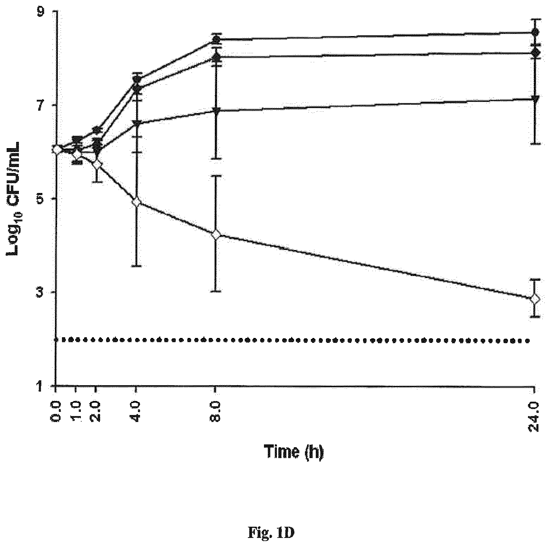

Figure 1 shows synergistic combinations (mean values) demonstrated with time-kill curves for clinical isolates (A) 2 ESBL-producing *E. coli,* (B) 2 ESBL-producing *K. pneumoniae,* (C) 2 AmpC-derepressed *E. cloacae* and (D) 3 *P. aeruginosa* isolates. The legends used are as follows: (-●-) Growth control, (-▼-) Ceftaroline, (-○-) Meropenem, (-Δ-) Ceftaroline plus Meropenem, (-■-) Piperacillin-Tazobactam (4/1), (-□-) Ceftaroline plus Piperacillin-Tazobactam, (-◆-) Amikacin, (-◊-) Ceftaroline plus Amikacin, (¨●¨) Aztreonam, (-○-) Ceftaroline plus Aztreonam and (...) Limit of detection.

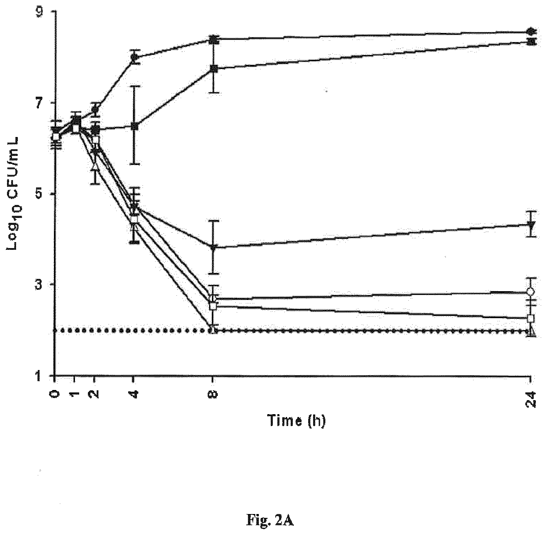

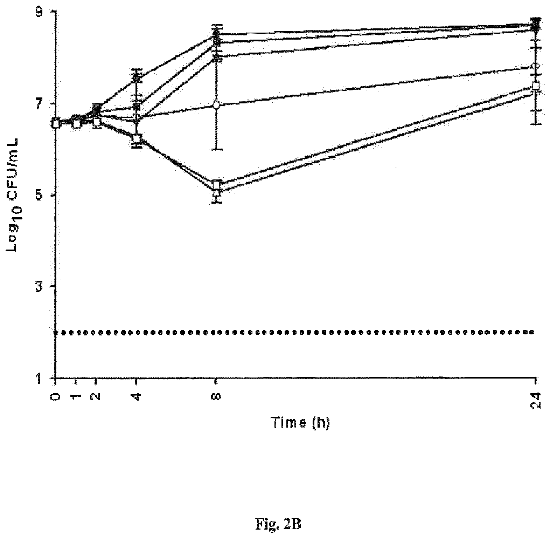

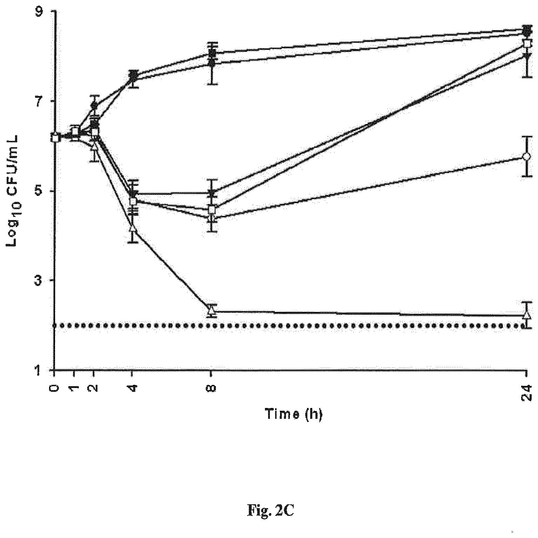

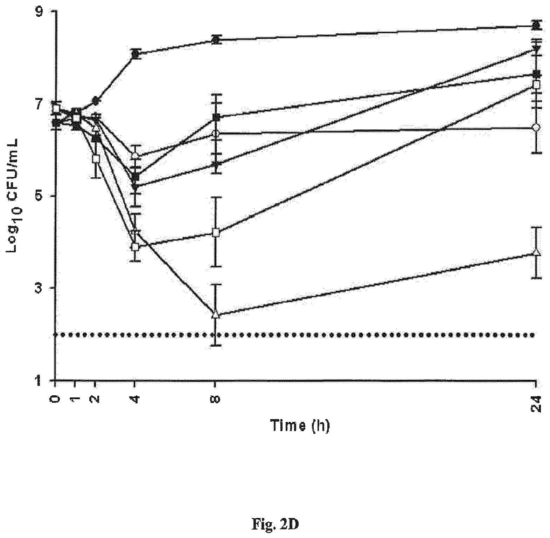

Figure 2 shows *in vitro* activity of ceftaroline, vancomycin and tobramycin alone or in combination at ½ MIC against 4 HA-MRSA. Results are presented as time-kill curves for (A) isolate R3875 (*h*VISA), (B) isolate R2303 (VISA), (C-D) isolates R3804 and R4039. The legends used are as follows: (●) Growth control, (○) Ceftaroline, (T) Tobramycin, (■) Vancomycin, (Δ) Ceftaroline plus Tobramycin, (□) Vancomycin plus Tobramycin and (...) Limit of detection.

### DETAILED DESCRIPTION OF THE INVENTION

The present invention provides compositions comprising ceftaroline fosamil, or a pharmaceutically acceptable salt or solvate thereof, and tobramycin, or a pharmaceutically acceptable salt thereof.

In exemplary embodiments, the compositions are for intravenous or intramuscular route of administration.

### Dosage Forms

The pharmaceutical composition includes, but is not limited to, dosage forms such as, tablets (including a sugar-coated tablet, a film-coated tablet), pills, capsules (including microcapsule), granules, fine granules, powders, drop infusions, syrups, emulsions, suspensions, injections, aerosols, suppositories, troches, cataplasms, ointments, gels, creams, sustained release preparations, etc. In exemplary embodiments, the dosage forms are suitable for intravenous or intramuscular route of administration.

These preparations can be prepared by a conventional method. As carriers for injectable preparations, use is made of, for example, distilled water or a physiological saline solution or any other suitable diluent. Carriers for capsules, powdery preparations, granular preparations or tablets are used as a mixture with known pharmaceutically acceptable excipients (for example, starch, maltose, sucrose, calcium carbonate or calcium phosphate), binders (for example, starch, gum arabic, carboxymethyl cellulose, hydroxypropyl cellulose or crystalline cellulose), lubricants (for example, magnesium stearate or talc) and disintegrants (for example, carboxymethyl calcium and talc).

In particular embodiments, the compositions may be presented in the form of a powder to be dissolved extemporaneously in an appropriate vehicle, for example, apyrogenic sterile water. The active ingredients may be incorporated with the excipients usually used in these pharmaceutical compositions, such as talc, gum arabic, lactose, starch, magnesium stearate, cocoa butter, aqueous or non aqueous vehicles, fatty matter of animal or vegetable origin, paraffin derivatives, glycols, various wetting, dispersing or emulsifying agents, preservatives.

In other embodiments, the pharmaceutical composition may comprise pharmaceutically acceptable carriers, including, but not limited to, diluents and bulking agents, which are selected from excipients, such as, calcium carbonate, kaolin, sodium hydrogen carbonate, lactose, D-mannitol, starch, crystalline cellulose, talc, fine granulated sugar and porous substance; binders, such as, dextrin, gums, α-starch, gelatin, hydroxypropylcellulose, hydroxy propyl methyl cellulose and pullulan; thickeners such as, natural gum, cellulose derivative, acrylic acid derivative; disintegrators, such as, carboxymethylcellulose calcium, crosscarmelose sodium, crospovidone, a low-substituted hydroxypropylcellulose and partly pregelatinized starch; solvents such as, water for injection, alcohol, propylene glycol, Macrogol, sesame oil and corn oil; dispersants, such as, Tween 80, HCO60, polyethylene glycol, carboxymethylcellulose, and sodium alginate; solubilizing agents, such as, polyethylene glycol, propylene glycol, D-mannitol, benzoic acid benzyl, ethanol, tris amino methane, triethanolamine, sodium carbonate, and citric acid sodium; suspending agents, such as, stearyl triethanolamine, sodium lauryl sulfate, benzalkonium chloride, polyvinyllcohol, and polyvinylpyrolidone, hydroxymethylcellulose; soothing agents, such as, benzyl alcohol; isotonic agents such as, sodium chloride and glycerin; buffer agents, such as, phosphoric acid salt, acetic acid salt, carbonic acid salt and citric acid salt; lubricants, such as, magnesium stearate, calcium stearate, talc, starch and sodium benzoate; coloring agents, such as, tar pigment, caramel, ferric oxide, titanium oxide and riboflavins; corrigents, such as, sweetning agents and perfumes; stabilizers, such as, sodium sulfite and ascorbic acid; and preservatives, such as, paraben and sorbic acid.

Numerous standard references are available that describe procedures for preparing various formulations suitable for administering the compounds according to the invention. Examples of potential formulations and preparations are contained, for example, in the Handbook of Pharmaceutical Excipients, American Pharmaceutical Association (current edition); Pharmaceutical Dosage Forms: Tablets (Lieberman, Lachman and Schwartz, editors) current edition, published by Marcel Dekker, Inc., as well as Remington's Pharmaceutical Sciences (Arthur Osol, editor), 1553-1593 (current edition). The mode of administration and dosage forms is closely related to the therapeutic amounts of the compounds or compositions which are desirable and efficacious for the given treatment application.

Suitable dosage forms include, but are not limited to oral, rectal, sub-lingual, mucosal, nasal, ophthalmic, subcutaneous, intramuscular, intravenous, transdermal, spinal, intrathecal, intra-articular, intra-arterial, sub-arachinoid, bronchial, lymphatic, and intra-uterille administration, and other dosage forms for systemic delivery of active ingredients. To prepare such pharmaceutical dosage forms, the active ingredient, is intimately admixed with a pharmaceutical carrier according to conventional pharmaceutical compounding techniques. The carrier may take a wide variety of forms depending on the form of preparation desired for administration.

In preparing the compositions in oral dosage form, any of the usual pharmaceutical media may be employed. Thus, for liquid oral preparations, such as, for example, suspensions, elixirs and solutions, suitable carriers and additives include water, glycols, oils, alcohols, flavoring agents, preservatives, coloring agents and the like. For solid oral preparations such as, for example, powders, capsules and tablets, suitable carriers and additives include starches, sugars, diluents, granulating agents, lubricants, binders, disintegrating agents and the like. If desired, tablets may be sugar coated or enteric coated by standard techniques.

For parenteral formulations, the carrier will usually comprise sterile water, though other ingredients, for example, ingredients that aid solubility or for preservation, may be included. Injectable solutions may also be prepared in which case appropriate stabilizing agents may be employed.

In some applications, it may be advantageous to utilize the active agent in a "vectorized" form, such as by encapsulation of the active agent in a liposome or other encapsulant medium, or by fixation of the active agent, e.g., by covalent bonding, chelation, or associative coordination, on a suitable biomolecule, such as those selected from proteins, lipoproteins, glycoproteins, and polysaccharides.

Formulations suitable for oral administration may be presented as discrete units such as capsules, cachets, tablets, or lozenges, each comprising a predetermined amount of the active ingredient as a powder or granules. Optionally, a suspension in an aqueous liquor or a non-aqueous liquid may be employed, such as a syrup, an elixir, an emulsion, or a draught.

A tablet may be made by compression or molding, or wet granulation, optionally with one or more accessory ingredients. Compressed tablets may be prepared by compressing in a suitable machine, with the active compound being in a free-flowing form such as a powder or granules which optionally is mixed with, for example, a binder, disintegrant, lubricant, inert diluent, surface active agent, or discharging agent. Molded tablets comprised of a mixture of the powdered active compound with a suitable carrier may be made by molding in a suitable machine.

A syrup may be made by adding the active compound to a concentrated aqueous solution of a sugar, for example sucrose, to which may also be added any accessory ingredient(s). Such accessory ingredient(s) may include flavorings, suitable preservative, agents to retard crystallization of the sugar, and agents to increase the solubility of any other ingredient, such as a polyhydroxy alcohol, for example glycerol or sorbitol.

Formulations suitable for parenteral administration usually comprise a sterile aqueous preparation of the active compound, which preferably is isotonic with the blood of the recipient (e.g., physiological saline solution). Such formulations may include suspending agents and thickening agents and liposomes or other microparticulate systems which are designed to target the compound to blood components or one or more organs. The formulations may be presented in unit-dose or multi-dose form.

Parenteral administration may be intravenous, intra-arterial, intrathecal, intramuscular, subcutaneous, intramuscular, intra-abdominal (e.g., intraperitoneal), etc., and may be effected by infusion pumps (external or implantable) or any other suitable means appropriate to the desired administration modality.

Nasal and other mucosal spray formulations (e.g. inhalable forms) can comprise purified aqueous solutions of the active compounds with preservative agents and isotonic agents. Such formulations are preferably adjusted to a pH and isotonic state compatible with the nasal or other mucous membranes. Alternatively, they can be in the form of finely divided solid powders suspended in a gas carrier. Such formulations may be delivered by any suitable means or method, e.g., by nebulizer, atomizer, metered dose inhaler, or the like.

Formulations for rectal administration may be presented as a suppository with a suitable carrier such as cocoa butter, hydrogenated fats, or hydrogenated fatty carboxylic acids.

Transdermal formulations may be prepared by incorporating the active agent in a thixotropic or gelatinous carrier such as a cellulosic medium, e.g., methyl cellulose or hydroxyethyl cellulose, with the resulting formulation then being packed in a transdermal device adapted to be secured in dermal contact with the skin of a wearer.

In addition to the aforementioned ingredients, formulations of this invention may further include one or more accessory ingredient(s) selected from diluents, buffers, flavoring agents, binders, disintegrants, surface active agents, thickeners, lubricants, preservatives (including antioxidants), and the like.

The formulations of the present invention can have immediate release, sustained release, delayed-onset release or any other release profile known to one skilled in the art.

### Methods of Treatment

The present invention provides compositions useful in treating a bacterial infection.

The bacterial infection may be due to Gram-positive bacteria, including, but not limited to, methicillin resistant *Staphylococcus aureus* (MRSA), community-acquired methicillin resistant *Staphylococcus aureus* (CAMRSA), vancomycin-intermediate-susceptible *Staphylococcus aureus* (VISA), methicillin-resistant coagulase-negative staphylococci (MR-CoNS), vancomycin-intermediate-susceptible coagulase-negative staphylococci (VI-CoNS), methicillin susceptible *Staphylococcus aureus* (MSSA), *Streptococcus pneumoniae* (including penicillin-resistant strains [PRSP]) and multidrug resistant strains [MDRSP]), *Streptococcus agalactiae, Streptococcus pyogenes* and *Enterococcus faecalis.* In other embodiments, the bacterial infection may be due to Gram-negative bacteria, such as, *Escherichia coli, Enterobacter cloacae, Klebsiella pneumoniae, Pseudomonas aeruginosa, Haemophilus influenzae* (including ampicillin-resistant *H. influenzae), Moraxella catarrhalis, Proteus mirabilis* and *Acinetobacter baumanii.*

The bacterial infection may be due to a microoraganism, including, but not limited to, *Citrobacter freundii, Citrobacter koseri, Enterobacter aerogenes, Enterobacter cloacae, Haemophilus parainfluenzae, Klebsiella oxytoca, Morganella morganii, Proteus vulgaris, Providencia rettgeri, Providencia stuartii, Serratia marcescens, Clostridium clostridioforme, Eubacterium lentum, Peptostreptococcus* species, *Porphyromonas asaccharolytica, Clostridium perfringens* and *Fusobacterium* species.

The bacterial infection may include, but is not limited to, complicated skin and skin structure infections (cSSSI); community acquired pneumonia (CAP); complicated intra-abdominal infections, such as, complicated appendicitis, peritonitis, complicated cholecystitis and complicated diverticulitis; uncomplicated and complicated urinary tract infections, such as, pyelonephritis; and respiratory and other nosocomial infections.

Ceftaroline, or a pharmaceutically acceptable salt or solvate thereof, and tobramycin, or a pharmaceutically acceptable salt thereof, may be administered in singular dose or may be administered in two to six divided doses for example, every 4 hours, 6 hours, 8 hours or 12 hours.

The bacterial infection may be a complicated skin and skin structure infection or a community acquired pneumonia.

Ceftaroline fosamil, or a pharmaceutically acceptable salt or solvate thereof and tobramycin, or a pharmaceutically acceptable salt thereof, may be administered in therapeutically effective dosages, which may vary according to the type of infection, the patient in question, the administration route and the antibacterial agent. They may be administered non-orally or orally, for example, as injectable preparations, capsules, tablets or granular preparations.

The intravenous or intramuscular route of administration may be used.

Ceftaroline fosamil, or a pharmaceutically acceptable salt or solvate thereof, and tobramycin, or a pharmaceutically acceptable salt thereof, may be administered in a combined dose of about 1 mg to 20 g/day in single or multiple administrations. The combined dose may range from about 10 mg to 10 g/day. The combined dose may range from about 20 mg to 5 g/day. The combined dose may range from about 30 mg to 2 g/day. The combined daily dose may be about 20 mg, 50 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2050 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2750 mg, 2800 mg, 2850 mg, 2900 mg, 2950 mg, 3000 mg, 3.5 g, 4 g, 4.5 g, 5 g, 5.5 g, 6 g, 6.5 g, 7 g, 7.5 g, 8 g, 8.5 g, 9 g, 9.5 g and 10 g.

Ceftaroline fosamil, or a pharmaceutically acceptable salt or solvate thereof, may be administered in a daily dose ranging from about 0.5 mg/kg to about 400 mg/kg, preferably from about 2 mg to 40 mg/kg of body weight of a man or an animal infected with pathogenic bacteria. The daily dose may range from about 5 to 30 mg/kg of body weight. The daily dose may be about 20 mg/kg of body weight. The daily dose may be administered in a singular dose, for example, every 24 hours. The daily dose may be administered in two to six divided doses, for example, every 4 hours, 6 hours, 8 hours or 12 hours.

Ceftaroline fosamil, or a pharmaceutically acceptable salt or solvate thereof, may be administered in doses ranging from about 1 mg to about 3000 mg per day in single or multiple administrations. Ceftaroline fosamil, or a pharmaceutically acceptable salt or solvate thereof, may be administered in single or multiple doses of about 10 mg, 20 mg, 30 mg, 40 mg, 50 mg, 100 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1050 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg and 1800 mg per day. For example, the daily dose of ceftaroline fosamil, or a pharmaceutically acceptable salt or solvate thereof, is about 400 mg, about 600 mg, about 800 mg or about 1200 mg.

About 400 mg of ceftaroline fosamil may be administered every 8 hours, 12 hours or 24 hours. About 600 mg of ceftaroline fosamil thereof may be administered every 8 hours, 12 hours or 24 hours. The duration of treatment is between five to seven days, five to ten days, or five to fourteen days.

The daily dose of tobramycin may range from about 0.001 to 20 mg/kg of body weight. The daily dose of tobramycin may be about 1 to 10 mg/kg of body weight. The daily dose of tobramycin may range from about 1 mg to 800 mg. The daily dose of tobramycin may range from about 10 mg to 600 mg. The daily dose of tobramycin may be about 1 mg, 2 mg, 5 mg, 10 mg, 15 mg, 20 mg, 30 mg, 40 mg, 50 mg, 60 mg, 70 mg, 80 mg, 90 mg, 100 mg, 110 mg, 120 mg, 130 mg, 140 mg, 150 mg, 160 mg, 170 mg, 180 mg, 190 mg, 200 mg, 250 mg, 275 mg, 300 mg, 325 mg, 350 mg, 375 mg, 400 mg, 425 mg, 450 mg, 475 mg, 500 mg, 525 mg, 550 mg, 575 mg and 600 mg.

The duration of treatment may depend on the type, severity and site of infection, the patient's clinical and bacteriological progress and the administration route. The treatment may last between five to fourteen days. The treatment may last between about five to ten days. The treatment may last between about five to seven days.

### Definitions

The term "pharmaceutically acceptable" means biologically or pharmacologically compatible for *in vivo* use in animals or humans, and preferably means approved by a regulatory agency of the Federal or a state government or listed in the U.S. Pharmacopeia or other generally recognized pharmacopeia for use in animals, and more particularly in humans.

The term "prodrug" means a compound that is a drug precursor, which upon administration to a subject undergoes chemical conversion by metabolic or chemical processes to yield a compound, which is an active moiety. Suitable prodrugs of ceftaroline include, but are not limited to phosphonocepehem derivatives, such as, *e.g.,* 7β-[2(Z)-ethoxyimino-2-(5-phosphonoamino-1,2,4-thiadiazol-3-yl)acetamido]-3-[4-(1-methyl-4-pyridinio)-2-thiazolythio]-3-cephem-4-carboxylate.

Solvates of a compound may form when a solvent molecule(s) is incorporated into the crystalline lattice structure of ceftaroline or a prodrug thereof molecule during, for example, a crystallization process. Suitable solvates include, *e.g.,* hydrates (monohydrate, sesquihydrate, dihydrate), solvates with organic compounds (*e.g.,* CH₃CO₂H, CH₃CH₂CO₂H, CH₃CN), and combinations thereof.

The terms "treat," "treatment," and "treating" refer to one or more of the following: relieving or alleviating at least one symptom of a bacterial infection in a subject; relieving or alleviating the intensity and/or duration of a manifestation of bacterial infection experienced by a subject; and arresting, delaying the onset (i.e., the period prior to clinical manifestation of infection) and/or reducing the risk of developing or worsening a bacterial infection.

An "effective amount" means the amount of a composition according to the invention that, when administered to a patient for treating an infection or disease is sufficient to effect such treatment. The "effective amount" will vary depending on the active ingredient, the state, infection, disease or condition to be treated and its severity, and the age, weight, physical condition and responsiveness of the mammal to be treated.

The term "therapeutically effective" applied to dose or amount refers to that quantity of a compound or pharmaceutical composition that is sufficient to result in a desired activity upon administration to a mammal in need thereof.

A subject or patient in whom administration of the therapeutic compound is an effective therapeutic regimen for an infection or disease is preferably a human, but can be any animal, including a laboratory animal in the context of a trial or screening or activity experiment. Thus, as can be readily appreciated by one of ordinary skill in the art, the compositions of the present invention are particularly suited to administration to any animal, particularly a mammal, and including, but by no means limited to, humans, domestic animals, such as feline or canine subjects, farm animals, such as, but not limited to, bovine, equine, caprine, ovine, and porcine subjects, wild animals (whether in the wild or in a zoological garden), research animals, such as mice, rats, rabbits, goats, sheep, pigs, dogs, cats, etc., avian species, such as chickens, turkeys, songbirds, etc., i.e., for veterinary medical use.

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviations, per practice in the art. Alternatively, "about" with respect to the compositions can mean plus or minus a range of up to 20%, preferably up to 10%, more preferably up to 5%. Alternatively, particularly with respect to biological systems or processes, the term can mean within an order of magnitude, preferably within 5-fold, and more preferably within 2-fold, of a value.

### EXAMPLES

The following examples are illustrative of the present invention and should not be construed as limiting the scope of the invention in any way.

### REFERENCE EXAMPLE 1

### Ceftaroline combinations using broth microdilution method

The activity of ceftaroline with other antimicrobials against target species was evaluated using a broth microdilution checkerboard technique. The broth microdilution checkerboard technique was used to generate fractional inhibitory concentration (FIC) and FIC index (FICI) values. The FICI of ceftaroline (CPT) in combination with vancomycin (VA), linezolid (LZD), levofloxacin (LVX), azithromycin (AZM), daptomycin (DAP), amikacin (AN), aztreonam (ATM), tigecycline (TGC), and meropenem (MEM) was determined against multiple isolates of clinically important target species using plates prepared in a semi-automated fashion.

### Material and Methods

Ceftaroline (ceftaroline fosamil; PPI-0903M; Lot No. M599-R1001) was provided by Cerexa, Inc. Other agents were obtained as follows: vancomycin (Lot No. 016K1102), amikacin (Lot No. 044K1473), aztreonam (Lot No. 124K1448), amoxicillin (Lot No. 112K0481), clavulanic acid (Lot No. 115K1493) and chloramphenicol (Lot No. 123K0588) were obtained from Sigma-Aldrich; azithromycin (Lot No. HOC212), meropenem (Lot No. GOF100) and ciprofloxacin (Lot No. IOC265) were obtained from USP; daptomycin (Lot No. CDX01#1007-1), levofloxacin (Lot No. 446423/1); linezolid (Lot No. LZD05003); tigecycline (Lot No. RB5603 Way 156936-9) were obtained from Cubist, Fluka, Pfizer and Wyeth respectively.

Stock solutions of all antibacterial agents were prepared at 80-fold (80X) the final target concentration in the appropriate solvent and the solution was allowed to stand for 60 minutes. All antibacterial agents were in solution under these conditions. The final drug concentrations in the FIC assay plates were set to bracket the MIC value of each agent for each test organism, unless the strain was totally resistant to the test agent. The concentration ranges tested are displayed in Table 1.

### Test Organisms

The test organisms were originally received from clinical sources, or from the American Type Culture Collection. Upon receipt, the isolates were streaked onto the appropriate growth medium: Chocolate Agar for *H. influenzae,* Tryptic Soy Agar II (Becton Dickinson, Sparks, MD) supplemented with 5% defibrinated sheep blood for streptococci, and unsupplemented Tryptic Soy Agar II for all other organisms. Colonies were harvested from these plates and a cell suspension was prepared in Tryptic Soy Broth (Becton Dickinson) containing cryoprotectant. Aliquots were then frozen at -80°C. On the day prior to assay, the frozen seeds of the organisms to be tested in that session were thawed and streaked for isolation onto the appropriate agar medium plates and incubated overnight at 35°C.

### Test Media

The test medium for *H. influenzae* was Haemophilus Test Medium. Streptococci were tested in Mueller Hinton II Broth (Becton Dickinson; Lot 6235472) supplemented with 2% lysed horse blood (Cleveland Scientific, Bath, OH; Lot H88621). All other organisms were tested in Mueller Hinton II Broth (Becton Dickinson, Lot 6235472). The broth was prepared at 1.05X normal weight/volume to offset the 5 % volume of the drugs in the final test plates.

### Minimal Inhibitory Concentration (MIC) Assay

In order to select the proper test concentrations for each drug combination, minimal inhibitory concentration (MIC) values were first determined using the broth microdilution method previously described (Clinical and Laboratory Standards Institute. Methods for Dilution Antimicrobial Susceptibility Tests for Bacteria That Grow Aerobically; Approved Standard-Seventh Edition. Clinical and Laboratory Standards Institute document M7-A7 [ISBN 1-56238-587-9]. Clinical and Laboratory Standards Institute, 940 West Valley Road, Suite 1400, Wayne, Pennsylvania 19087-1898 USA, 2006).

### FIC Assay Methodology

FIC values were determined using a broth microdilution method previously described (Sweeney and Zurenko, 2003; Antimicrob. Agents Chemother. 47:1902-1906). Automated liquid handlers (Multidrop 384, Labsystems, Helsinki, Finland; Biomek 2000 and Multimek 96, Beckman Coulter, Fullerton CA) were used to conduct serial dilutions and liquid transfers.

The wells of standard 96-well microdilution plates (Falcon 3918) were filled with 150µL of 100% DMSO using the Multidrop 384. These plates were used to prepare the drug "mother plates" which provided the serial drug dilutions for the drug combination plates. The Biomek 2000 was used to transfer 150 µl of each stock solution (80X) from the wells in Column 1 of a deep well plate to the corresponding wells in Column 1 of the mother plate and to make seven 2-fold serial dilutions. Two mother plates, one for each drug, were combined to form a "checkerboard" pattern by transfer of equal volumes (using a multi-channel pipette) to the drug combination plate. Row H and Column 8 each contained serial dilutions of one of the agents alone for determination of the MIC.

The "daughter plates" were loaded with 180 µL of test medium using the Multidrop 384. Then, the Multimek 96 was used to transfer 10 µL of drug solution from each well of the drug combination mother plate to each corresponding well of the daughter plate in a single step. Finally, the daughter plates were inoculated with test organism. Standardized inoculum of each organism was prepared per published guidelines (Clinical and Laboratory Standards Institute. Methods for Dilution Antimicrobial Susceptibility Tests for Bacteria That Grow Aerobically; Approved Standard-Seventh Edition. Clinical and Laboratory Standards Institute document M7-A7 [ISBN 1-56238-587-9]. Clinical and Laboratory Standards Institute, 940 West Valley Road, Suite 1400, Wayne, Pennsylvania 19087-1898 USA, 2006). The inoculum for each organism was dispensed into sterile reservoirs divided by length (Beckman Coulter), and the Biomek 2000 was used to inoculate the plates. The instrument delivered 10 µL of standardized inoculum into each well to yield a final cell concentration in the daughter plates of approximately 5 x 10⁵ colony-forming-units/mL.

The test format resulted in the creation of an 8 x 8 checkerboard where each compound was tested alone (Column 8 and Row H) and in combination at varying ratios of drug concentration. Assay reproducibility was monitored using *S. aureus* 0100 and the combination of amoxicillin-clavulanate, which yields a synergistic result with this test strain due to its β-lactamase-positive status. Chloramphenicol and quinolones are recognized as a combination that may be antagonistic. Accordingly, the combination of chloramphenicol and ciprofloxacin was tested to demonstrate either no interaction or an antagonistic interaction of a drug combination. On two of the assay dates, an additional strain (*E. faecalis* 0101) was tested with chloramphenicol-ciprofloxacin.

Plates were stacked 3 high, covered with a lid on the top plate, placed in plastic bags, and incubated at 35°C for approximately 20 hours. Following incubation, the microplates were removed from the incubator and viewed from the bottom using a ScienceWare plate viewer. Prepared reading sheets were marked for the MIC of drug 1 (row H), the MIC of drug 2 (column 8) and the wells of the growth-no growth interface.

### FIC Calculations

The FIC was calculated as: (MIC of Compound 1 in combination/MIC of Compound 1 alone) + (MIC of Compound 2 in combination/MIC of Compound 2 alone). The FIC index (FICI) for the checkerboard was calculated from the individual FICs by the formula: (FIC₁ + FIC₂ + ... FICₙ)/n, where n = number of individual wells per plate for which FICs were calculated. In instances where an agent alone yielded an off-scale MIC result, the next highest concentration was used as the MIC value in the FIC calculation.

FICI values have been interpreted in a variety of ways (Eliopoulos and Moellering, 1991; Antimicrobial combinations. In Antibiotics in Laboratory Medicine, Third Edition, edited by V Lorian. Williams and Wilkins, Baltimore, MD, 432-492). Most commonly, FICI values have been defined as follows: ≤ 0.50, synergism; >0.50-2, indifference; >2, antagonism. More recently (Odds, 2003; J. Antimicrob. Chemother. 52(1):1), FICI values have been interpreted as follows. A "synergistic interaction" was evidenced by inhibition of organism growth by combinations that are at concentrations significantly below the MIC of either compound alone, resulting in a low FICI value (≤0.50). The interpretation of "no interaction" results in growth inhibition at concentrations below the MICs of the individual compounds, but the effect is not significantly different from the additive effects of the two compounds, resulting in an FICI value of >0.50 but less than or equal to 4.0. The interpretation "no interaction" has previously been referred to as "additivity" or "indifference". An "antagonistic interaction" results when the concentrations of the compounds in combination that are required to inhibit organism growth are greater than those for the compounds individually, resulting in an FIC value of >4.0. Thus, while the definition of synergism has remained constant, the definition of additivity/indifference has been broadened and re-named to "no interaction". In addition, the FICI value indicative of antagonism has been re-defined as >4. While there is no officially-sanctioned set of FICI criteria, the literature has been consistent in the use of ≤0.50 to define synergism.

### Results

The test concentrations for each pair of test agents for each test organism are shown in Table 1. All of the agents alone or in combination were soluble at all final test concentrations. Several control drug combinations were included in each FIC assay (Table 2). The control combination of amoxicillin and clavulanic acid demonstrated the expected synergistic interaction (FICI value ≤0.50) for the control organism *S. aureus* 0100 in all FIC assays. The control combination of chloramphenicol and ciprofloxacin, which was expected to demonstrate a negative interaction for *S. aureus* or *E. faecalis,* yielded relatively high FICI values that would be categorized as either antagonism or no interaction, depending upon the FICI cut-off criteria applied.

The MIC and FICI values are shown in Tables 3 to 11. The interpretation listed in the tables for each test organism and drug combination is based upon the recently published FICI criteria (Odds, 2003). The combination of ceftaroline and vancomycin, linezolid, daptomycin, and tigecycline (Tables 3, 4, 5, and 6, respectively) yielded a result of no interaction for the staphylococci, enterococci, and streptococci, and Gram-negative organisms tested. For the combination of ceftaroline and meropenem (Table 7), two instances of synergy were detected (*S. aureus* 2202 and *K pneumoniae* 1468), and there was no interaction for the rest of the test organisms. Ceftaroline in combination with levofloxacin (Table 8) yielded a result of no interaction for a broad range of Gram-positive and Gram-negative organisms. The combination of ceftaroline and amikacin (Table 9) resulted in two instances of synergy [*E. coli* 2273 (ESBL) and *P. aeruginosa* 2559], and notably, the FICI values for all other strains tested were < 1. Ceftaroline combined with aztreonam (Table 10) demonstrated no interaction for all of the strains tested, though three of the strains had relatively low FICI values. The combination of ceftaroline and azithromycin (Table 11) yielded no interaction for pneumococci and *H. influenzae.*

The testing of ceftaroline in combination with various antibacterial agents against individual representative bacterial strains surprisingly and unexpectedly demonstrated several incidences of synergism and a result of no interaction for all of the other organisms tested. Furthermore, no evidence of antagonism was observed for the drug combinations tested. Thus, ceftaroline may be successfully combined with an antibacterial agent to provide compositions for the treatment of bacterial infections.

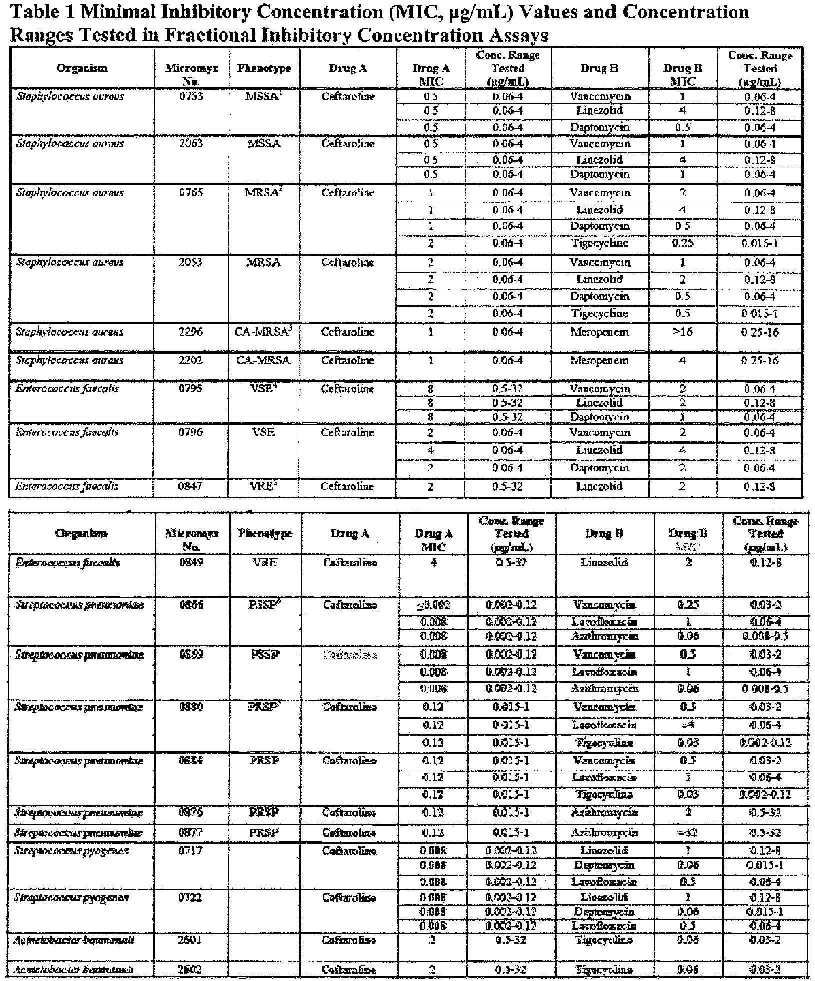

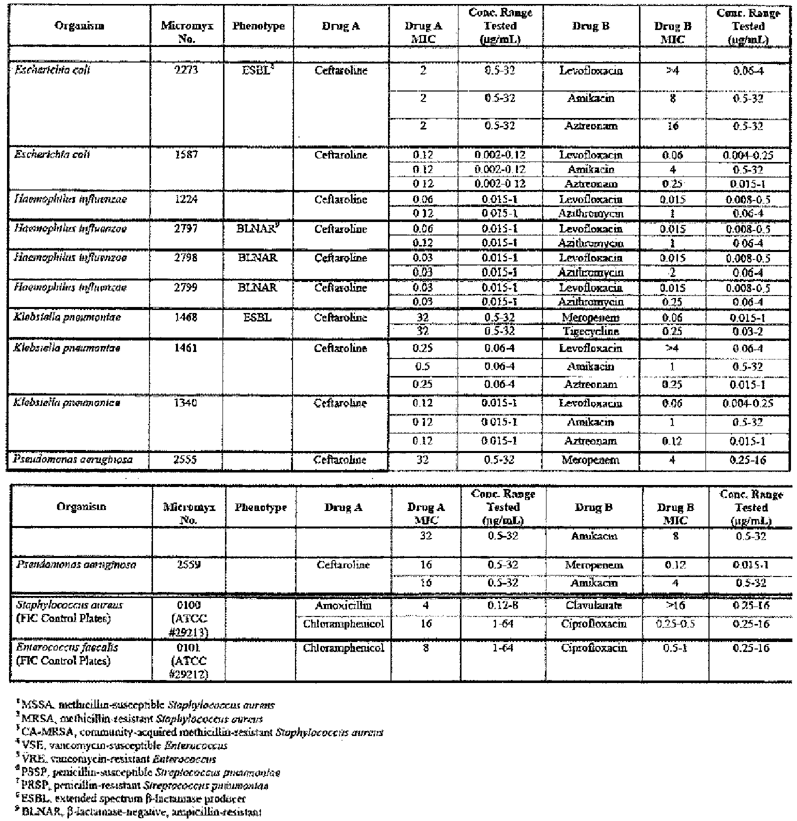

**Table 2 Summary of Control Results for the Combinations Amoxicillin-Clavulanic Acid and Chloramphenicol-Ciprofloxacin**

| | **Compound 1** | | **Compounds 2** | | |
|---|---|---|---|---|---|
| **Organism** | **Name** | **MIC¹ (µg/mL) Alone** | **Name** | **MIC (µg/mL) Alone** | **FICI²** |
| *S. aureus* 0100 | | | | | 0.25 |
| (ATCC 29213) | Amoxicillin | 4 | Clavulanate | > 16 | 0.27 |
| | | | | | 0.25 |
| | | | | | 0.28 |
| | | | | | |
| *S. aureus* 0100 | | | | 0.50 | 2.07 |
| (ATCC 29213) | Chloramphenicol | 16 | Ciprofloxacin | 0.50 | 2.78 |
| | | | | 0.50 | 1.91 |
| | | | | 0.25 | 3.32 |
| | | | | | |
| *E. faecalis* 0101 | | | | 1 | 2.66 |
| (ATCC 29212) | Chloramphenicol | 8 | Ciprofloxacin | 0.5 | 3.23 |
| | | | | 1 | 1.53 |
| ¹MIC, Minimum Inhibitory Concentration | | | | | |
| ²FICI, Fractional Inhibitory Concentration Index | | | | | |

**Table 3 Summary of Minimum Inhibitory Concentration and Fractional Inhibitory Concentration Results for Ceftaroline and Vancomycin**

| | **Compound 1** | | **Compound 2** | | | |
|---|---|---|---|---|---|---|
| **Organism (Phenotype ¹)** | **Name** | **MIC² (µg/mL) Alone** | **Name** | **MIC (µg/mL) Alone** | **FICI³** | **Interpretation** |
| *S. aureus* 0753 (MSSA) | | 0.5 | | 1 | 1.05 | No Interaction |
| *S. aureus* 2063 (MSSA) | | 0.5 | | 1 | 0.96 | No Interaction |
| *S. aureus* 0765 (MRSA) | | 1 | | 2 | 1.17 | No Interaction |
| *S. aureus* 2053 (MRSA) | | 2 | | 1 | 138 | No Interaction |
| *E. faecalis* 795 (VSE) | Ceftaroline | 8 | Vancomycin | 2 | 1.05 | No Interaction |
| *E. faecalis* 796 (VSE) | | 2 | | 2 | 1.34 | No Interaction |
| *S. pneumoniae* 866 (PSSP) | | ≤0.002 | | 0.25 | 2.95 | No Interaction |
| *S. pneumoniae* 869 (PSSP) | | 0.008 | | 0.5 | 0.79 | No Interaction |
| *S. pneumoniae* 880 (PRSP) | | 0.12 | | 0.5 | 0.86 | No Interaction |
| *S. pneumoniae* 884 (PRSP) | | 0.12 | | 0.5 | 0.66 | No Interaction |
| Footnotes for Tables 3 -11 : | | | | | | |
| ¹ Phenotype: MSSA, methicillin-susceptible *Staphylococcus aureus* ; MRSA, methicillin-resistant *Staphylococcus aureus* ; CA-MRSA, community-acquired methicillin-resistant *Staphylococcus aureus*; VSE, vancomycin-susceptible *Enterococcus* VRE, vancomycin-resistant *Enterococcus* ; PSSP, penicillin-susceptible *Streptococcus pneumoniae*; PRSP, penicillin-resistant *Streptococcus pneumoniae* ; ESBL, extended-spectrum β-lactamase producer; BLNAR, β-lactamase-negative, ampicillin resistant | | | | | | |
| ²MIC, Minimum Inhibitory Concentration | | | | | | |
| ³FICI, Fractional Inhibitory Concentration Index | | | | | | |
| ⁴FICI interpretation ≤ 0.5, synergism; > 4, antagonism; > 0.5 to 4.0, no interaction | | | | | | |

**Table 4 Summary of Minimum Inhibitory Concentration and Fractional Inhibitory Concentration Results for Ceftaroline and Linezolid**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. aureus* 0753 (MSSA) | | 0.5 | | 4 | 0.80 | No Interaction |
| *S. aureus* 2063 (MSSA) | | 0.5 | | 4 | 0.72 | No Interaction |
| *S. aureus* 0765 (MRSA) | | 1 | | 4 | 1.11 | No Interaction |
| *S. aureus* 2053 (MRSA) | | 2 | | 2 | 1.05 | No Interaction |
| *E. faecalis* 795 (VSE) | | 8 | | 2 | 1.23 | No Interaction |
| *E. faecalis* 796 (VSE) | Ceftaroline | 4 | Linezolid | 4 | 0.77 | No Interaction |
| *E. faecalis* 847 (VRE) | | 2 | | 2 | 1.14 | No Interaction |
| *E. faecalis* 849 (VRE) | | 4 | | 2 | 1.26 | No Interaction |
| *S. pyogenes* 717 | | 0.008 | | 1 | 1.32 | No Interaction |
| *S. pyogenes* 722 | | 0.008 | | 1 | 1.32 | No Interaction |

**Table 5 Summary of Minimum Inhibitory Concentration and Fractional Inhibitory Concentration Results for Ceftaroline and Daptomycin**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. aureus* 0753 (MSSA) | | 0.5 | | 0.5 | 0.93 | No Interaction |
| *S. aureus* 2063 (MSSA) | | 0.5 | | 1 | 0.70 | No Interaction |
| *S. aureus* 0765 (MRSA) | | 1 | | 0.5 | 0.96 | No Interaction |
| *S. aureus* 2053 (MRSA) | Ceftaroline | 2 | Daptomycin | 0.5 | 0.72 | No Interaction |
| *E. faecalis* 795 (VSE) | | 8 | | 1 | 0.57 | No Interaction |
| *E. faecalis* 796 (VSE) | | 2 | | 2 | 0.63 | No Interaction |
| *S. pyogenes* 717 | | 0.008 | | 0.06 | 0.75 | No Interaction |
| *S. pyogenes* 722 | | 0.008 | | 0.06 | 1.17 | No Interaction |

**Table 6 Summary of Minimum Inhibitory Concentration and Fractional Inhibitory Concentration Results for Ceftaroline and Tigecycline**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. aureus* 0765 (MRSA) | | 2 | | 0.25 | 1.05 | No Interaction |
| *S. aureus* 2053 (MRSA) | | 2 | | 0.5 | 1.10 | No Interaction |
| *S. pneumoniae* 880 (PRSP) | | 0.12 | | 0.03 | 1.42 | No Interaction |
| *S. pneumoniae* 884 (PRSP) | Ceftaroline | 0.12 | Tigecycline | 0.03 | 1.26 | No Interaction |
| | | | | | | |
| *K. pneumoniae* 1468 (ESBL) | | 32 | | 0.25 | 1.36 | No Interaction |
| *A. baumannii* 2601 | | 2 | | 0.06 | 1.42 | No Interaction |
| *A. baumannii* 2602 | | 2 | | 0.06 | 1.42 | No Interaction |

**Table 7 Summary of Minimum Inhibitory Concentration and Fractional Inhibitory Concentration Results for Ceftaroline and Meropenem**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. aureus* 2296 (CA-MRSA) | | 1 | | > **16^{a}** | 0.62 | No Interaction |
| *S. aureus* 2202 (CA-MRSA) | | 1 | | 4 | 0.44 | Synergy |
| | | | | | | |
| *K. pneumoniae* 1468 (ESBL) | Ceftaroline | 32 | Meropenem | 0.06 | 0.49 | Synergy |
| *P. aeruginosa* 2555 | | 32 | | 4 | 0.60 | No Interaction |
| *P. aeruginosa* 2559 | | 16 | | 0.12 | 1.65 | No Interaction |
| ^{a} Value of 32 µg/mL used for FIC calculation | | | | | | |

**Table 8 Summary of Minimum Inhibitory Concentration and Fractional Inhibitory Concentration Results for Ceftaroline and Levofloxacin**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. pneumoniae* 866 (PSSP) | | 0.008 | | 1 | 1.14 | No Interaction |
| *S. pneumoniae* 869 (PSSP) | | 0.008 | | 1 | 1.04 | No Interaction |
| *S. pneumoniae* 880 (PRSP) | | 0.12 | | >4^{a} | 1.19 | No Interaction |
| *S. pneumoniae* 884 (PRSP) | | 0.12 | | 1 | 1.13 | No Interaction |
| *S. pyogenes* 717 | | 0.008 | | 0.5 | 1.15 | No Interaction |
| *S. pyogenes* 722 | | 0.008 | | 0.5 | 0.90 | No Interaction |
| | | | | | | |
| *K. pneumoniae* 1461 | Ceftaroline | 0.25 | Levofloxacin | > 4^{a} | 1.75 | No Interaction |
| *K. pneumoniae* 1340 | | 0.12 | | 0.06 | 1.14 | No Interaction |
| *E. coli* 2273 (ESBL) | | 2 | | > 4^{a} | 1.86 | No Interaction |
| *E. coli* 1587 | | 0.12 | | 0.06 | 1.05 | No Interaction |
| *H. influenzae* 1224 | | 0.06 | | 0.015 | 1.76 | No Interaction |
| *H. influenzae* 2797 (BLNAR) | | 0.06 | | 0.015 | 1.43 | No Interaction |
| *H. influenzae* 2798 (BLNAR) | | 0.03 | | 0.015 | 1.52 | No Interaction |
| *H. influenzae* 2799 (BLNAR) | | 0.03 | | 0.015 | 1.52 | No Interaction |
| ^{a} Value of 8 µg/mL used for FIC calculation | | | | | | |

**Table 9 Summary of Minimum Inhibitory Concentration and Fractional Inhibitory Concentration Results for Ceftaroline and Amikacin**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *K. pneumoniae* 1461 | | 0.5 | | 1 | 0.79 | No Interaction |
| *K. pneumoniae* 1340 | | 0.12 | | 1 | 0.96 | No Interaction |
| *E. coli* 2273 (ESBL) | Ceftaroline | 2 | Amikacin | 8 | 0.50 | Synergy |
| *E. coli* 1587 | | 0.12 | | 4 | 0.96 | No Interaction |
| *P. aeruginosa* 2555 | | 32 | | 8 | 0.83 | No Interaction |
| *P. aeruginosa* 2559 | | 16 | | 4 | 0.42 | Synergy |

**Table 10 Summary of Minimum Inhibitory Concentration and Fractional Inhibitory Concentration Results for Ceftaroline and Aztreonam**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *K. pneumoniae* 1461 | Ceftaroline | 0.25 | Aztreonam | 0.25 | 1.27 | No Interaction |
| *K. pneumoniae* 1340 | | 0.12 | | 0.12 | 0.83 | No Interaction |
| *E. coli* 2273 (ESBL) | | 2 | | 16 | 0.64 | No Interaction |
| *E. coli* 1587 | | 0.12 | | 0.25 | 0.60 | No Interaction |

**Table 11 Summary of Minimum Inhibitory Concentration and Fractional Inhibitory Concentration Results for Ceftaroline and Azithromycin**

| | **Compound 1** | | **Compound 2** | | | |
|---|---|---|---|---|---|---|
| **Organism (Phenotype)** | **Name** | **MIC (µg/mL) Alone** | **Name** | **MIC (µg/mL) Alone** | **FICI** | **Interpretation** |
| *S. pneumoniae* 866 (PSSP) | | 0.008 | | 0.06 | 1.16 | No. Interaction |
| *S. pneumoniae* 869 (PSSP) | | 0.008 | | 0.06 | 1.16 | No Interaction |
| *S. pneumoniae* 876 (PRSP) | | 0.12 | | 2 | 1.11 | No Interaction |
| *S. pneumoniae* 877 (PRSP) | | 0.12 | | >32^{a} | 0.99 | No Interaction |
| | Ceftaroline | | Azithromycin | | | |
| *H. influenzae* 1224 | | 0.12 | | 1 | 1.26 | No Interaction |
| *H. influenzae* 2797 (BLNAR) | | 0.12 | | 1 | 1.13 | No Interaction |
| *H. influenzae* 2798 (BLNAR) | | 0.03 | | 2 | 1.24 | No Interaction |
| *H. influenzae* 2799 (BLNAR) | | 0.03 | | 0.25 | 1.11 | No Interaction |
| ^{a} Value of 64 µg/mL used for FIC calculation | | | | | | |

### REFERENCE EXAMPLE 2

### Ceftaroline combinations using time kill curve method

The *in vitro* activity of ceftaroline combined with meropenem, piperacillin-tazobactam, cefepime, amikacin, levofloxacin, aztreonam and tigecycline was evaluated. Susceptibility testing was performed for 20 clinical *P. aeruginosa,* 10 ESBL-producing *Escherichia coli,* 10 ESBL-producing *Klebsiella pneumoniae* and 10 AmpC-derepressed *Enterobacter cloacae.* Time-kill experiments were run for 10 randomly selected isolates with antimicrobials at ¼ MIC.

### Materials and Methods

### Bacterial strains

Twenty clinical *P. aeruginosa* from the Anti-infective Research Laboratory (ARL, Detroit, MI, USA), 10 ESBL-producing *E, coli,* 10 ESBL-producing *Klebsiella pneumoniae,* as well as 10 AmpC-derepressed *Enterobacter cloacae* were selected from ARL and JMI Laboratories (North Liberty, IA, USA) clinical isolate collections for susceptibility testing. Ten strains (2 *E. coli, 2 K. pneumoniae, 2 E. cloacae* and 4 *P. aeruginosa*) with various susceptibility levels for ceftaroline were randomly selected to be run in time-kill experiments.

### Antimicrobial agents

Ceftaroline (ceftaroline fosamil) was provided by Cerexa, Inc (Alabama, CA, USA). Piperacillin, tazobactam, tigecycline (Wyeth Pharmaceuticals, Inc., Pearl River, NY, USA), meropenem (AstraZeneca Pharmaceuticals LP, Wilmington, DE, USA) and cefepime (Elan Pharmaceuticals, Inc., San Diego, CA, USA) were commercially purchased. Levofloxacin, amikacin and aztreonam were obtained from Sigma-Aldrich Co. (St Louis, MO, USA).

### Medium

Mueller-Hinton broth (MHB; Difco Laboratories, Detroit, MI, USA) supplemented with magnesium (12.5 µg/mL total concentration) and calcium (25 µg/mL total concentration) (SMHB) was used for all microdilution susceptibility testing and time-kill analysis. Tryptose soya agar (TSA; Difco Laboratories, San Jose, CA, USA) was used for growth and to quantify colony counts.

### Susceptibility testing

Minimum inhibitory concentrations (MICs) as well as minimum bactericidal concentrations (MBCs) of the tested drugs were determined using broth microdilution methods according to clinical and laboratory institute (CLSI) guidelines (Clinical and Laboratory Standards Institute. 2006. Methods for Dilution Antimicrobial Susceptibility Tests for Bacteria That Grow Aerobically; Approved Standard. 7th ed. Wayne, PA: CLSI) All susceptibility testing were performed in duplicate, at a starting inoculum of ∼5.5 x 10⁵ CFU/mL and concentrations ranged up to 1024 µg/mL for ceftaroline alone or combined to tazobactam (4/1); up to 256 µg/mL for amikacin and tigecycline and up to 64 µg/mL for aztreonam, meropenem, cefepime, piperacillin/tazobactam and levofloxacin.

### Time-kill curve analysis

Time-kill experiments were performed in duplicate with an initial inoculum of ∼10⁶ CFU/mL. Ten randomly chosen strains, including 2 *E. coli, 2 K. pneumoniae, 2 E. cloacae* and 4 *P. aeruginosa,* harboring various susceptibility levels for ceftaroline, were exposed to each tested drug alone or in combination at ¼ MIC. Regimens included aztreonam, meropenem, cefepime, amikacin, piperacillin/tazobatam, levofloxacin, tigecycline and ceftaroline alone or combined to each of the listed antimicrobials. Aliquots (0.1 mL) were removed from cultures at 0, 1, 2, 4, 8 and 24-h and serially diluted in cold 0.9% sodium chloride. Synergy, additive effect and indifference were defined as >2 log₁₀ kill, <2 but >1 log₁₀ kill and ±1 log kill, respectively, compared to the most efficient agent at 24-h. Antagonism was defined as >1 log10 growth compared with the least active single agent at 24-h. Bacterial counts were determined by spiral plating appropriate dilutions using an automatic spiral plater (WASP; DW Scientific, West Yorkshire, UK) and by counting colonies using the protocol colony counter (Synoptics Limited, Frederick, MD, USA). The lower limit of detection for colony count was 2 log₁₀ CFU/mL. Time-kill curves were constructed by plotting mean colony counts (log₁₀ CFU/mL) versus time. Bactericidal activity of drug alone was defined as a ≥3 log₁₀ CFU/mL (99.9 %) reduction at 24-h from the starting inoculum although bactericidal activity of drug combination was defined as a ≥3 log₁₀ CFU/mL (99.9 %) reduction compared to the most efficient drug at 24-h.

### Results

Except for 4 *E. cloacae* (MIC ≤ 1), ceftaroline exhibited a MIC range of 2-1024 µg/mL, reduced from 2 to 128 fold by combination with tazobactam for ESBL-producing strains. In time-kill experiments, no antimicrobial alone was bactericidal. Combinations of ceftaroline plus tigecycline, levofloxacin or cefepime were mainly indifferent. Whereas, ceftaroline plus amikacin was synergistic for 9 isolates, ceftaroline plus piperacillin-tazobactam was synergistic for *E. coli* and *K. pneumoniae,* indifferent for *E. cloacae* and indifferent/additive for *P. aeruginosa.* Ceftaroline plus meropenem or aztreonam was synergistic for *E. coli* and *E. cloacae* respectively, but indifferent against all other isolates, except 1 *P. aeruginosa* (additivity). No antagonism was observed with any combination. Ceftaroline in combination with amikacin appeared synergistic against 90% of the tested strains.

### Susceptibility

Selected clinical Enterobacteriaceae represented a large panel of strains, harboring various susceptibility levels for ceftaroline and other tested antimicrobials (Table 12). Ceftaroline MIC values ranged from 2 to 1024 µg/mL. According to the ceftaroline susceptibility break points recently proposed (Brown and Traczewski, 2007; Abstr. D-240, 47th Intersci. Conf. Antimicrob. Agents Chemother.) selected isolates included: 8 susceptible strains (MIC ≤4 µg/mL): 3 *E. coli,* 1 *K. pneumoniae, 4 E. cloacae* ; 8 intermediate strains (MIC = 8 µg/mL): 2 *E. coli,* 2 *K. pneumoniae* and 4 *P. aeruginosa,* and 34 resistant strains (MIC ≥ 16µg/mL): 5 *E. coli, 7 K. pneumoniae, 6 E. cloacae* and 16 *P. aeruginosa.* In combination with tazobactam (in proportion of 4/1), ceftaroline MIC was decreased from 2 to 128-fold for ESBL-producing *E. coli* and *K. pneumoniae* strains. Thus, 9 *E. coli* isolates became susceptible and 1 exhibited intermediate susceptibility to ceftaroline. For the *K. pneumoniae* isolates, 6 isolates were susceptible to ceftaroline after addition of tazobactam, 2 were intermediate and only 2 strains still exhibited resistance to ceftaroline (but with a decrease of MIC of 8 and 16-fold). Addition of tazobactam decreased ceftaroline MIC 2-fold for some AmpC-derepressed *E. cloacae* and for some *P. aeruginosa* isolates (Table 12). Thus, tazobactam did not change the susceptibility profile of *E. cloacae* and *P. aeruginosa* strains, which still were resistant or intermediate. MBC values of ceftaroline (alone or combined to tazobactam) were found similar or one dilution higher than MIC values (Table 12). Other antimicrobials exhibited various levels of susceptibility against selected clinical strains, with MIC ranges from 0.03 to ≥ 32 µg/mL. However, Enterobacteriacae appeared susceptible to meropenem and tigecycline with MIC values ≤4 and 8 µg/mL, respectively. All tested *P. aeruginosa* were susceptible to amikacin with a MIC range of 2 to 16 µg/mL. Furthermore, except 1 isolate (MIC = 0.5 µg/mL), all *K. pneumoniae* were resistant to aztreonam, with MIC values ≥ 8 µg/mL (Table 12).

**Table 12 Susceptibility profiles (MIC/MBC ranges) of the 30 tested clinical Enterobacteriacae and 20 P. aeruginosa isolates**

| Antimicrobials | MIC/MBC ranges (µg/mL) | | | |
|---|---|---|---|---|
| | *E. coli* (10) | *K. pneumonia* (10) | *E. cloacae* (10) | *P. aeruginosa* (20) |
| Ceftaroline | 2 - 512 / 4-1024 | 8 - 1024 / 32-1024 | 0.125 - 512 / 0.125-1024 | 8 - 256 / 16-256 |
| Ceftaroline-Tazobactam (4/1) | 1 - 8 / 1 - 16 | 1 - 64 / 4-64 | 0.125 - 256 / 0.125 - 256 | 8 - 128 / 16-256 |
| Meropenem | 0.03-0.06/0.06 -0.125 | 0.63 - 0.06 / 0.06 - 0.125 | 0.03 - 0.25 / 0.03 - 0.5 | 0.125 - 16 / 0.125 - 32 |
| Cefepime | 0.125 - 256 / 0.25 - 512 | 0.5 - 16 / 0.5->64 | 0.06 - 32 / 0.125 - 64 | 2 - 32/4 - 64 |
| Piperacillin-Tazobactam (4/1) | 2 - 64 / 2 - 128 | 2 ->256 / 4 - >256 | 2 - 128 / 4 - 256 | 2 - 256/4-256 |
| Aztreonam | 0.125 - >64 / 0.125 - >64 | 0.25 - >64 / 0.5 - >64 | 0.125 - >64 / 0.125 - >64 | 2 - 64/8 - 64 |
| Amikacin | 1 - 16 / 4 - 64 | 1 - 32 / 1 - 128 | 0.5 - 4 / 1 - 8 | 2 - 16/2 - 64 |
| Levofloxacin | 0.03 - 32 / 0.06 - 32 | 0.25 - >32 / 0.25 - >32 | 0.03 - 64 / 0.03 - 128 | 0.25 - 32 / 0.5 - >32 |
| Tigecycline | 0.06 - 0.5 / 0.06 - 4 | 0.125 - 1 / 0.5 - 8 | 0.25 - 2 / 0.5 - 4 | 2 - 32 / 8 - 256 |

### Time-kill analysis

Potential of synergy was evaluated for 10 randomly selected isolates, harboring various susceptibility levels for each tested antimicrobials, including ceftaroline (Tables 13 and 14). In time-kill experiments, ceftaroline and the other agents alone were not bactericidal at ¼ MIC. In combination, ceftaroline with tigecycline, levofloxacin and cefepime were mainly indifferent (mean decrease from 0.01 to 0.20 ± 0.30 log₁₀ CFU/mL). Additive effect was demonstrated with ceftaroline plus levofloxacin against *K. pneumoniae* isolate n. 5427 (mean decrease at 1.7 ± 0.20 log₁₀ CFU/mL). Combination of ceftaroline plus cefepime was additive against 1 *P. aeruginosa* (isolate n.1037), with a decrease of 1.8 ± 0.40 log₁₀ CFU/mL. In contrast, ceftaroline plus amikacin demonstrated synergistic effect against all tested strains. Mean differences were ∼ 5.65, 4.4, 5.1 and 3.6 log₁₀ CFU/mL for *E. coli, E. cloacae, K. pneumoniae* and *P. aeruginosa,* respectively (Figure 1). Ceftaroline combined with meropenem, aztreonam or piperacillin-tazobactam led to various antimicrobial effects. Ceftaroline plus piperacillin-tazobactam (4/1) was synergistic against both *E. coli* and *K. pneumoniae* isolates, with similar mean differences (∼ 5.82 and 5.33 log₁₀ CFU/mL) (Figures 1a-b). In contrast, ceftaroline plus piperacillin-tazobactam (4/1) was indifferent against the 2 *E. cloacae* isolates (5417 and 4073) and 1 *P. aeruginosa* (isolate n. 956). An additive effect was observed with *P. aeruginosa* isolate n. 1037, with ceftaroline plus piperacilin-tazobactam (1.81 ± 0.42 log₁₀ CFU/mL) as well as plus aztreonam (1.01 ± 0.54 log₁₀ CFU/mL). Finally, combination of ceftaroline with meropenem was synergistic against ESBL producing *E. coli* (∼ 4.45 log₁₀ CFU/mL), as well as ceftaroline plus aztreonam against AmpC-derepressed *E. cloacae* isolates (∼ 3.03 log₁₀ CFU/mL) (Figures 1a and 1c). No antagonism was observed in the study.

Several drug combinations surprisingly and unexpectedly extended ceftaroline broad-spectrum of activity to most of MDR Gram-negative organisms. Several antimicrobials led to synergistic effect in combination with ceftaroline.

**Table 13 In vitro activity of ceftaroline and tested antimicrobials (MIC/MBC) against the 10 selected clinical isolates**

| | MIC/MBC (µg/mL) | | | | | | | | | |
|---|---|---|---|---|---|---|---|---|---|---|
| | *E. coli* | | *K. pneumonia* | | *E. cloacae* | | *P. aeruginosa* | | | |
| | Isolate no. | | Isolate no. | | Isolate no. | | Isolate no. | | | |
| | 5401 | 5411 | 5427 | 5436 | 4073 | 5420 | 796 | 956 | 1019 | 1037 |
| Ceftaroline | 4/8 | 64/128 | 4/16 | 1024/1024 | 256/512 | 64/128 | 16/32 | 128/256 | 32/64 | 8/16 |
| Ceftaroline-Tazobactam (4/1) | 1/2 | 0.5/4 | 1/1 | 8/8 | 256/256 | 64/128 | 8/16 | 64/128 | 32/64 | 4/16 |
| Meropenem | 0.06/0.06 | 0.06/0.06 | 0.06/0.06 | 0.06/0.06 | 0.25/0.5 | 0.125/0.125 | 1/2 | 0.25/2 | 1/2 | 0.5/1 |
| Cefepime | 4/4 | 2/4 | 0.5/1 | 16/32 | 4/16 | 0.25/1 | 8/16 | 8/32 | 2/4 | 1/2 |
| Piperacillin-Tazobactam (4/1) | 64/128 | 16/32 | 2/4 | 4/4 | 64/64 | 64/64 | 4/16 | 4/32 | 4/8 | 4/16 |
| Aztreonam | 0.25/0.25 | 8/32 | 0.5/1 | 64/64 | 32/64 | 8/16 | 4/32 | 8/64 | 4/4 | 4/8 |
| Amikacin | 2/4 | 8/16 | 2/2 | 1/2 | 1/8 | 1/2 | 16/32 | 4/64 | 4/4 | 2/4 |
| Levoflox acin | 32/32 | 8/16 | 0.25/2 | 4/4 | 0.06/0.06 | 0.06/0.06 | 1/2 | 0.5/1 | 0.5/1 | 0.25/0.5 |
| Tigecycli ne | 0.5/1 | 0.125/0.5 | 0.5/2 | 0.125/1 | 0.5/2 | 0.5/2 | 32/32 | 16/128 | 2/8 | 8/32 |

**Table 14 In vitro activity of combinations against the 10 randomly selected clinical isolates**

| Drug combinations | Species | Isolate | Decrease of the bacterial count (mean log₁₀ ± SD) at: | | | | Effect |
|---|---|---|---|---|---|---|---|
| | | | 2-h | 4-h | 8-h | 24-h | |
| Ceftaroline + Meropenem | *E. coli* | 5401 | 0.87 ± 0.20 | 3.12 ± 0.14 | 4.20 ± 0.72 | 4.93 ± 0.75 | S |
| | 5411 | 5411 | 0.08 ± 0.58 | 1.78 ± 0.15 | 3.91 ± 0.13 | 4.17 ± 0.47 | S |
| | *K. pneumoniae* | 5427 | 0.06 ± 0.13 | 1.13 ± 0.46 | 0.12 ± 0.05 | 0.12 ± 0.03 | I |
| | | 5436 | 0.59 ± 0.05 | 1.16 ± 0.15 | 3.66 ± 0..32 | 0.04 ± 0.12 | I |
| | *E. cloacae* | 4073 | 0.86 ± 0.13 | 1.39 ± 0.28 | 1.21 ± 0.09 | 0.72 ± 0.20 | I |
| | | 5420 | 0.10 ± 0.02 | 1.03 ± 0.73 | 1.44 ± 0.74 | 0.12 ± 0.11 | I |
| | *P. aeruginosa* | 796 | 0.07 ± 0.08 | 0.03 ± 0.04 | 0.05 ± 0.03 | 0.14 ± 0.16 | I |
| | | 956 | 0.02 ± 0.03 | 0.21 ± 0.27 | 0.10 ± 0.01 | 0.31 ± 0.31 | I |
| | | 1019 | 0.01 ± 0.01 | 0.27 ± 0.10 | 0.04 ± 0.05 | 0.05 ± 001 | I |
| | | 1037 | 0.20 ± 0.04 | 0.32 ± 0.27 | 0.28 ± 0.15 | 1.71 ± 0.14 | I |
| Ceftaroline + Piperacillin-Tazobactam | *E. coli* | 5401 | 0.29 ± 0.01 | 2.24 ± 0.12 | 4.49 ± 0.16 | 5.79 ± 0.57 | S |
| | | 5411 | 0.81 ± 0.58 | 2.88 ± 0.58 | 4.38 ± 0.81 | 5.85 ± 0.40 | S |
| | | 5427 | 0.10 ± 0.00 | 1.52 ± 0.18 | 4.11 ± 0.07 | 5.39 ± 0.62 | S |
| | | 5436 | 0.26 ± 0.36 | 0.87 ± 0.13 | 3.31 ± 0.01 | 5.28 ± 0.11 | S |
| | *E. cloacae* | 4073 | 0.04 ± 0.02 | 0.04 ± 0.16 | 0.17 ± 0.19 | 0.02 ± 0.02 | I |
| | | 5420 | 0.15 ± 0.15 | 0.80 ± 0.74 | 0.08 ± 0.10 | 0.04 ± 0.05 | I |
| | *P. aeruginosa* | 796 | 0.52 ± 0.31 | 0.17 ± 0.17 | 0 08 ± 0.02 | 0.00 ± 0.06 | I |
| | | 956 | 0.00 ± 0.20 | 0.19 ± 0.10 | 0.05 ± 0.10 | 0.10 ± 0.06 | I |
| | | 1019 | 0.07 ± 0.03 | 0.07 ± 0.23 | 0.01 ± 0.10 | 0.12 ± 0.00 | I |
| | | 1037 | 0.52 ± 0.18 | 1.17± 0.19 | 1.66 ± 0.47 | 1.81 ± 0.42 | A |
| Ceftaroline + Amikacin | *E. coli* | 5401 | 1.01 ± 0.00 | 4.78 ± 0.08 | 5.30 ± 0.08 | 5.32 ± 0.02 | S |
| | | 5411 | 0.93 ± 0.80 | 2.88 ± 0.32 | 5.10 ± 0.06 | 5.98 ± 0.37 | S |
| | | 5427 | 0.65 ± 0.24 | 3.42 ± 0.08 | 4.43 ± 0.38 | 4.81 ± 0.34 | S |
| | | 5436 | 0.26 ± 0.36 | 0.87 ± 0.14 | 3.31 ± 0.01 | 5.31 ± 0.14 | S |
| | *E. cloacae* | 4073 | 1.38 ± 0.01 | 1.68 ± 0.01 | 3.19 ± 0.16 | 4.65 ± 0.02 | S |
| | | 5420 | 0.02 ± 0.02 | 1.12 ± 0.80 | 3.27 ± 0.36 | 4.44 ± 0.72 | S |
| | *P. aeruginosa* | 796 | 0.07 ± 0.05 | 0.63 ± 0.31 | 2.14 ± 0.41 | 5.23 ± 0.32 | S |
| | | 956 | 0.36 ± 0.05 | 2.61 ± 0.02 | 2.66 ± 0.50 | 3.60 ± 0.44 | S |
| | | 1019 | 0.16 ± 0.11 | 1.32 ± 0.07 | 3.84 ± 0.81 | 0.67 ± 0.28 | I |
| | | 1037 | 0.09 ± 0.08 | 2.13 ± 0.07 | 2.71 ± 0.30 | 3.51 ± 0.27 | S |
| Ceflaroline + Levofloxacin | *E. coli* | 5401 | 0.11 ± 0.11 | 1.00 ± 0.21 | 0.05 ± 0.02 | 0.04 ± 0.06 | I |
| | | 5411 | 0.63 ± 0.58 | 0.58 ± 0.01 | 0.08 ± 0.14 | 0.01 ± 0.05 | I |
| | *K. pneumoniae* | 5427 | 0.15 ± 0.05 | 0.18 ± 0.07 | 1.68 ± 0.22 | 1.7 ± 0.20 | A |
| | | 5436 | 0.09 ± 0.07 | 1.05 ± 0.09 | 0.47 ± 0.08 | 0.08 ± 0.01 | I |
| | *E. cloacae* | 4073 | 0.10 ± 0.07 | 0.24 ± 0.02 | 0.10 ± 0.02 | 0.16 ± 0.01 | I |
| | | 5420 | 0.01 ± 0.10 | 0.41 ± 0.38 | 0.05 ± 0.14 | 0.04 ± 0.06 | I |
| | *P. aeruginosa* | 796 | 0.03 ± 0.02 | 0.21 ± 0.42 | 0.35 ± 0.09 | 0.04 ± 0.14 | I |
| | | 956 | 0.08 ± 0.09 | 0.25 ± 0.23 | 0.14 ± 0.00 | 0.98 ± 0.06 | I |
| | | 1019 | 0.21 ± 0.15 | 0.03 ± 0.01 | 1.21 ± 0.13 | 0.00 ± 0.00 | I |
| | | 1037 | 0.02 ± 0.10 | 0.10 ± 0.15 | 0.04 ± 0.01 | 0.10 ± 0.36 | I |
| Ceftaroline + Aztreonam | *E. coli* | 5401 | 0.32 ± 0.06 | 1.36 ± 0.01 | 0.12 ± 0.05 | 0.08 ± 0.01 | I |
| | | 5411 | 0.63 ± 0.25 | 0.99 ± 0.04 | 0.16 ± 0.03 | 0.33 ± 0.08 | I |
| | *K. pneumoniae* | 5427 | 0.01 ± 0.07 | 0.64 ± 0.02 | 0.06 ± 0.08 | 0.02 ± 0.09 | I |
| | | 5436 | 0.44 ± 0.01 | 0.37 ± 0.02 | 2.00 ± 0.09 | 0.14 ± 0.07 | I |
| | *E. cloacae* | 4073 | 0.69 ± 0.32 | 1.71± 0.25 | 1.73 ± 0.29 | 3.08 ± 0.13 | S |
| | | 5420 | 0 03 ± 0.04 | 0.90 ± 0.88 | 3.33 ± 0.91 | 2.99 ± 0.12 | S |
| | *P. aeruginosa* | 796 | 0.06 ± 0.11 | 0.04 ± 0.26 | 0.97 ± 0.18 | 0.85 ± 0.15 | I |
| | | 956 | 0.18 ± 0.15 | 0.12 ± 0.17 | 0.20 ± 0.53 | 0.73 ± 0.68 | I |

| Drug combinations | Species | Isolate | Decrease of the bacterial count (mean log₁₀ ± SD) at: | | | | Effect |
|---|---|---|---|---|---|---|---|
| | | | 2-h | 4-h | 8-h | 24-h | |
| | | 1019 | 0.03 ± 0.05 | 0.15 ± 0.22 | 0.15 ± 0.03 | 0.26 ± 0.27 | I |
| | | 1037 | 0.17 ± 0.09 | 0.12 ± 0.36 | 0.22 ± 0.16 | 1.01 ± 0.54 | A |
| Ceftaroline+ Tigecycline | *E. coli* | 5401 | 0.12 ± 0.08 | 0.45 ± 0.78 | 0.02 ± 0.11 | 0.13 ± 0.07 | I |
| | | 5411 | 0.23 ± 0.11 | 0.17 ± 0.09 | 0.08 ± 0.15 | 0.01 ± 0.02 | I |
| | *K. pneumoniae* | 5427 | 0.32 ± 0.07 | 0.59 ± 0.02 | 0.62 ± 0.59 | 0.25 ± 0.31 | I |
| | | 5436 | 0.13 ± 0.16 | 0.40 ± 0.52 | 0.22 ± 0.17 | 0.03 ± 0.02 | I |
| | *E. cloacae* | 4073 | 0.05 ± 0.06 | 0.43 ± 0.00 | 0.95 ± 0.00 | 0.14 ± 0.03 | I |
| | | 5420 | 0.13 ± 0.11 | 0.15 ± 0.11 | 0.08 ± 0.08 | 0.10 ± 0.12 | I |
| | *P. aeruginosa* | 796 | 0.05 ± 0.03 | 0.48 ± 0.08 | 0.01 ± 0.03 | 0.17 ± 0.28 | I |
| | | 956 | 0.12 ± 0.06 | 0.22 ± 0.28 | 0.28 ± 0.07 | 0.33 ± 0.38 | I |
| | | 1019 | 0.00 ± 0.02 | 0.10 ± 0.11 | 0.11 ± 0.01 | 0.14 ± 0.16 | I |
| | | 1037 | 0.10 ± 0.03 | 0.06 ± 0.31 | 1.07 ± 0.25 | 0.54 ± 0.12 | I |
| Ceftaroline + Cefepime | *E. coli* | 5401 | 0.37 ± 0.04 | 1.54 ± 0.12 | 0.04 ± 0.01 | 0.03 ± 0.02 | I |
| | | 5411 | 0.56 ± 0.51 | 0.38 ± 0.01 | 0.07 ± 0.00 | 0.01 ± 0.00 | I |
| | *K. pneumoniae* | 5427 | 0.08 ± 0.03 | 0.82 ± 0.19 | 0.38 ± 0.32 | 0.20 ± 0.30 | I |
| | | 5436 | 0.01 ± 0.07 | 0.51 ± 0.11 | 0.1.58 ± 0.06 | 0.31 ± 0.01 | I |
| | *E. cloacae* | 4073 | 0.17 ± 0.12 | 0.57 ± 0.14 | 0.55 ± 0.14 | 0.03 ± 0.02 | I |
| | | 5420 | 0.41 ± 0.33 | 0.12 ± 1.09 | 0.03 ± 0.06 | 0.07 ± 0.11 | I |
| | *P. aeruginosa* | 796 | 0.02 ± 0.04 | 0.27 ± 0.30 | 0.08 ± 0.01 | 0.02 ± 0.01 | I |

| Drug combinations | Species | Isolate | Decrease of the bacterial count (mean log₁₀ ± SD) at: | | | | Effect |
|---|---|---|---|---|---|---|---|
| | | | 2-h | 4-h | 8-h | 24-h | |
| | | 956 | 0.25 ± 0.24 | 0.01 ± 0.19 | 0.35 ± 0.37 | 0.52± 0.38 | I |
| | | 1019 | 0.13 ± 0.11 | 0.08 ± 0.08 | 0.03 ± 0.01 | 0.05± 0.29 | I |
| | | 1037 | 0.52 ± 0.18 | 1.17 ± 0.19 | 1.66 ± 0.47 | 1.81 ± 0.41 | A |

### Example 3

### In vitro activity and aminoglycoside synergy of ceftaroline against hospital acquired MRSA

The *in vitro* activity of ceftaroline and its potential for synergy in combination with tobramycin against a collection of hospital-acquired MRSA recovered from various clinical samples and exhibiting different level of resistance for vancomycin was evaluated.

### Materials and Methods

### Bacterial strains

Two hundred clinical HA-MRSA isolates, harboring the SCC*mec*IV type, were evaluated for susceptibility testing. All isolates, selected from the Anti-Infective Research Laboratory (ARL, Detroit, MI) collection, were isolated from patients at the Detroit Medical Center and were previously characterized on a molecular basis. Four strains, including 1 *h*VISA and 1 VISA, characterized by population analysis profile and Macro Etest were selected for time-kill analysis.

### Antimicrobial agents

Ceftaroline (ceftaroline fosamil) was provided by Cerexa Inc. Linezolid, vancomycin and tobramycin were commercially purchased (Pfizer Inc., New York, NY and Sigma Chemical Company, St Louis, MO, respectively).

### Media

Except for daptomycin, Mueller-Hinton broth (Difco, Detroit, MI) supplemented with calcium (25 mg/L) and magnesium (12.5 mg/L) (SMHB) was used for all susceptibility testing and time-kill experiments. For daptomycin experiments, SMHB was supplemented with 50 mg/L calcium and 12.5 mg/L magnesium. Trypose soy agar (TSA; Difco, Detroit, MI) was used for colony counting.

### Susceptibility testing

Minimum inhibitory concentrations (MIC) and minimum bactericidal concentrations (MBC) were determined by broth microdilution for all antimicrobials, according to the Clinical and Laboratory Standards Institute (CLSI) guidelines (Clinical and Laboratory Standards Institute. 2006. Methods for Dilution Antimicrobial Susceptibility Tests for Bacteria That Grow Aerobically; Approved Standard. 7th ed. Wayne, PA: CLSI). MBC values were determined by plating of aliquots of 5 µL from clear wells onto TSA. All susceptibility testing were performed in duplicate.

### Time-kill curves

Four randomly selected HA-MRSA isolates were evaluated in time-kill experiments, using a starting inoculum at ∼10⁶ CFU/mL and antimicrobials at ¼ and ½ MIC. Regimens included ceftaroline, vancomycin and tobramycin alone or combination of tobramycin with ceftaroline or vancomycin. Briefly, aliquots (0.1 mL) were removed from cultures at 0, 1, 2, 4, 8 and 24-h and serially diluted in cold 0.9% sodium chloride. Appropriate dilutions were plated using an automatic spiral plater (WASP; DW Scientific, West Yorkshire, UK) and bacterial counts were achieved using the protocol colony counter (Synoptics Limited, Frederick, MD, USA). Time-kill curves were constructed by plotting mean colony counts (log₁₀ CFU/mL) versus time. The lower limit of detection for colony count was 2 log₁₀ CFU/mL. Synergy was defined as a ≥2 log₁₀ CFU/mL increase in kill in comparison with the most effective antimicrobial alone at 24-h; bactericidal activity was defined as a ≥3 log₁₀ CFU/mL reduction at 24 h from the starting inoculum. Additivity, antagonism and indifference were defined as <2 but >1 log₁₀ kill, >1 log₁₀ growth and ±1 log kill, respectively.

### Statistical analysis

Differences between regimens were analyzed by T-test or ANOVA with Tukey's post hoc test. All statistical analysis was performed using SPSS statistical software (Release 15.0, SPSS, Inc., Chicago, IL). A P value < 0.05 was considered significant.

### Results

Ceftaroline was efficient against the collection of 200 HA-MRSA isolates recovered from various clinical samples. Susceptibility values and origin of the isolates are reported in Tables 15 and 16, respectively. Based on the CLSI susceptibility breakpoints and breakpoints recently proposed for ceftaroline (Brown and Traczewski, 2007 ; Program and Abstracts of the forty-seven Interscience Conference on Antimicrobial Agents and Chemotherapy, Chicago, IL, USA, 2007. Abstract D-239), all HA-MRSA, except one strain (isolate R2303), were susceptible to all tested antimicrobials. For vancomycin and linezolid, MIC values were similar and ranged from 0.25 to 4 µg/mL. MIC₅₀ and MIC₉₀ were one-fold higher for linezolid compared to vancomycin (1 and 2 µg/mL for vancomycin *versus* 2 and 4 µg/mL for linezolid, respectively). Daptomycin MIC range was lower (0.125 to 2 µg/mL), with MIC₅₀ at 0.25 µg/mL and MIC₉₀ at 0.5 µg/mL. MBC values were similar to the MICs, except for linezolid, which exhibited a MBC range from 0.5 to 64 µg/mL (Table 15).

Ceftaroline exhibited MIC values ranging from 0.25 to 4 µg/mL, with MIC₅₀ and MIC₉₀ at 1 µg/mL. A slight variability was observed since only 4 % of the strains exhibited an MIC at 0.25 µg/mL and 1.5 % at 2 µg/mL. MBC values were equal or one time higher than MICs (Table 15). Among the 200 isolates, 36 % were recovered from respiratory tract samples, 17 % from blood, 13.5 % from skin and 2 % from urine (Table 16). Thirty one percent were uncharacterized due to a lack of clinical information. Isolates removed from urine exhibited a lower MIC₅₀ value at 0.5 µg/mL, but the number of isolates in that group was not sufficient to make a reliable statement. Therefore, no difference was found in MIC values regarding the specimen sites as well as the susceptibility to vancomycin.

### Time-kill analysis and potential for synergy

Four HA-MRSA were selected to be run in time-kill experiments, using ceftaroline and vancomycin alone or combined with tobramycin. Two of these isolates (R2303 and R3578) presented a reduced susceptibility to vancomycin, and were previously characterized VISA (vancomycin MIC at 4 µg/mL) and hVISA (vancomycin MIC at 2 µg/mL). Two others HA-MRSA, susceptible to vancomycin, were randomly selected. MIC and MBC of these 4 strains are reported in Table 17. In this study, MBC values were found equal or one time higher than MICs (Table 15). Bactericidal activity was therefore closed to the inhibitory concentration. To assess to ceftaroline potential for synergy, time-kill experiments were therefore performed at ¼ and ½ MIC.

Since no activity as well as no synergy, additivity, antagonism or indifference was found in time-kill experiments at ¼ MIC, the results are reported at ½ MIC. Under those experimental conditions, none of the tested antimicrobial alone was bactericidal, except ceftaroline which displayed a persistent bactericidal activity against the hVISA (isolate R3875) (Figure 2a). Against the 2 vancomycin-susceptible MRSA, ceftaroline exhibited a lower activity compared to the *h*VISA, with 1.5 log₁₀ kill at 4 hours, followed by a bacterial regrowth (Figures 2c-d). Same phenomenon was observed with ceftobiprole, which demonstrated a potent killing activity alone at ½ MIC, against CA- (community-acquired) and HA- MRSA (Leonard and Rybak, 2008; Antimicrob Agents Chemother 52: 2974-2976). In contrast, vancomycin did not display bactericidal activity against none of the tested strains, including those without reduced susceptibility, and appeared therefore less efficient than ceftaroline alone (Figures 2a-d).

In combination with tobramycin at ½ MIC, a synergistic effect was observed with ceftaroline against both vancomycin-susceptible MRSA (isolates R3804 and R4039), with a bactericidal activity at 6.1 and 4.8 hours, respectively (Figures 2a-b). Vancomycin plus tobramycin was indifferent and the difference of activity between vancomycin plus tobramycin and ceftaroline plus tobramycin was statistically significant with *P* values of 0.001 against R3804 and 0.006 against R4039 (Figures 2a-b). Vancomycin plus tobramycin was synergistic against the *h*VISA isolate, demonstrating bactericidal activity at 5.8 hours. However, activity of the combination appeared non significantly different to that of ceftaroline alone or combined with tobramycin (P value of 0.061) (Figure 2c). Neither tested antimicrobials alone nor combinations with tobramycin demonstrated bactericidal activity or synergy effect against the VISA strain R2303 (Figure 2b).

**Table 15 Repartition of Minimal Inhibitory/Bactericidal Concentrations of 200 HA-MRSA isolates**

| | % of Isolates | | | | | | | | | | | | | |
|---|---|---|---|---|---|---|---|---|---|---|---|---|---|---|
| | MIC (µg/mL) | | | | | | Range | MBC (µg/mL) | | | | | | Range |
| | 0.125 | 0.25 | 0.5 | 1 | 2 | ≥4 | | 0.125 | 0.2 5 | 0.5 | 1 | 2 | ≥4 | |
| Ceftaroline | - | 4 | 35.5 | 57 | 1.5 | - | 0.25-2 | - | - | 22 | 62.5 | 15.5 | - | 0.5-2 |
| Vancomycin | - | 1.5 | 14.5 | 62 | 21.5 | 0.5 | 0.25-4 | - | 0.5 | 6 | 52.5 | 36.5 | 4.5 | 0.25-4 |
| Daptomycin | 19.5 | 58.5 | 20.5 | 1 | - | 0.5 | 0.125-4 | 9.5 | 47.5 | 35 | 5 | 2.5 | 0.5 | 0.125-4 |
| Linezolid | - | 9.5 | 16 | 15. 5 | 47. 5 | 11. 5 | 0.25-4 | - | - | 1. 5 | 12 | 13. 5 | 73 | 0.5-64 |

**Table 16 Susceptibility results for ceftaroline in function of the origins of the isolates**

| Origin | Number of isolates | Susceptibility (µg/mL) | | | |
|---|---|---|---|---|---|
| | | MIC₅₀ | Range | MBC₅₀ | Range |
| Skin^{a} | 27 | 1 | 0.5 - 2 | 1 | 0.5 - 2 |
| Lung^{b} | 72 | 1 | 0.25 - 2 | 1 | 0.5 - 2 |
| Blood^{c} | 34 | 1 | 0.25 - 2 | 1 | 0.5 - 2 |
| Urine | 4 | 0.5 | 0.5 - 1 | 1 | 0.5 - 2 |
| Unknown | 63 | 1 | 0.5 - 1 | 1 | 0.5 - 2 |
| ^{a}abscess, tissue and swab samples | | | | | |
| ^{b}aspirate, bronchial washing, lavage, endotracheale secretion and sputum | | | | | |
| ^{c}blood and catheter | | | | | |

**Table 17 Susceptibility results for 4 HA-MRSA isolates (including 1 hVISA and 1 VISA) selected to be run in time-kill experiments**

| Isolate n. | Susceptibility (µg/mL) | | | | | |
|---|---|---|---|---|---|---|
| | Ceftaroline | | Vancomycin | | Tobramycin | |
| | MIC | MBC | MIC | MBC | MIC | MBC |
| R2303 | 0.25 | 0.25 | 4 | 8 | 0.125 | 0.5 |
| R3875 | 1 | 1 | 2 | 2 | 0.5 | 0.5 |
| R3804 | 0.25 | 0.25 | 0.5 | 1 | 0.5 | 1 |
| R4039 | 0.5 | 0.5 | 1 | 1 | 1 | 1 |

## Claims

1. A pharmaceutical composition comprising a therapeutically effective amount of ceftaroline fosamil or a pharmaceutically acceptable salt or solvate thereof, and tobramycin or a pharmaceutically acceptable salt thereof.

2. The composition according to claim 1, wherein the composition comprises ceftaroline fosamil monoacetate monohydrate.

3. The composition according to claim 1, wherein the ceftaroline fosamil is anhydrous.

4. A composition according to any one of claims 1 to 3 for use in the treatment of a bacterial infection.

## Patentansprüche

1. Pharmazeutische Zusammensetzung, die eine therapeutisch wirksame Menge an Ceftarolinfosamil oder einem pharmazeutisch annehmbaren Salz oder Solvat davon und von Tobramycin oder einem pharmazeutisch annehmbaren Salz davon umfasst.

2. Zusammensetzung gemäß Anspruch 1, wobei die Zusammensetzung Ceftarolinfosamilmonoacetatmonohydrat umfasst.

3. Zusammensetzung gemäß Anspruch 1, wobei das Ceftarolinfosamil wasserfrei ist.

4. Zusammensetzung gemäß einem der Ansprüche 1 bis 3 zur Verwendung in der Behandlung einer bakteriellen Infektion.

## Revendications

1. Composition pharmaceutique comprenant une quantité thérapeutiquement efficace de ceftaroline fosamil ou d'un sel ou solvate pharmaceutiquement acceptable de celle-ci, et de la tobramycine ou un sel pharmaceutique acceptablement de celle-ci.

2. Composition selon la revendication 1, dans laquelle la composition comprend du monohydrate de monoacétate de ceftaroline fosamil.

3. Composition selon la revendication 1, dans laquelle la ceftaroline fosamil est anhydre.

4. Composition selon l'une quelconque des revendications 1 à 3 pour une utilisation dans le traitement d'une infection bactérienne.
